(19) Europäisches Patentamt
European Patent Office
Office européen des brevets

(11) Numéro de publication : **0 049 186**
**B1**

(12) # FASCICULE DE BREVET EUROPÉEN

(45) Date de publication du fascicule du brevet : **13.07.83**

(51) Int. Cl.³ : **C 07 C 121/52**, C 07 C 120/00

(21) Numéro de dépôt : **81401437.9**

(22) Date de dépôt : **16.09.81**

(54) **Procédé de préparation de fluorobenzonitriles.**

(30) Priorité : **25.09.80 FR 8020573**

(43) Date de publication de la demande :
**07.04.82 Bulletin 82/14**

(45) Mention de la délivrance du brevet :
**13.07.83 Bulletin 83/28**

(84) Etats contractants désignés :
**BE CH DE FR GB IT LI NL SE**

(56) Documents cités :
**EP A 0 001 825**
**EP A 0 005 090**
**EP A 0 005 094**
**FR A 2 391 990**

**CHEMISCHE BERICHTE, année 112, 1979 Verlag Chemie, GmbH Weinheim, DE U. HEIMANN et al. : « Ligandstruktur und Komplexierung, XXXVII 1) « Dreiarmige, multizähnige Neutralliganden — Synthese, Komplexstabilität und Kationselektivität » pages 1392-1399**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Ramanadin**
**Montée de la Daude**
**F-30100-Ales (FR)**
Inventeur : **Roustan, Alain**
**566, Chemin de Saint-Georges**
**F-30100-Ales (FR)**
Inventeur : **Soula, Gérard**
**33, rue Nungesser**
**F-69330-Meyzieu (FR)**

(74) Mandataire : **Cazes, Jean-Marie et al**
**RHONE-POULENC RECHERCHES**
**Service Brevets Chimie et Polymères**
**25, quai Paul Doumer**
**F-92408 Courbevoie Cedex (FR)**

EP 0 049 186 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation de fluorobenzonitriles

La présente invention a pour objet un procédé de préparation de fluorobenzonitriles. Elle concerne plus particulièrement la préparation de fluorobenzonitriles à partir des chlorobenzonitriles correspondants par réaction de ces derniers avec un fluorure alcalin.

Il est connu dans l'art antérieur de préparer des fluorobenzonitriles par réaction de chlorobenzonitriles avec un fluorure alcalin comme le fluorure de potassium dans des solvants aprotiques, comme par exemple le diméthylformamide, le diméthylsulfone et le sulfolane (voir « par exemple la demande de brevet britannique de 2.016.000) éventuellement en présence d'un catalyseur comme le fluorure de caesium (demande de brevet français 2.391.990).

La demanderesse a découvert un nouveau procédé qui permet d'augmenter très sensiblement la vitesse de réaction et qui permet donc d'améliorer la productivité. Ceci est d'une grande importance au plan industriel car l'homme de l'art sait bien l'intérêt que présente les fluorobenzonitriles en tant qu'intermédiaires de synthèse de composés ayant une activité phytosanitaire ou pharmaceutique.

La présente invention a donc pour objet un procédé de préparation de fluorobenzonitriles par réaction des chlorobenzonitriles correspondants avec au moins un fluorure alcalin en milieu solvant aprotique caractérisé en ce que la réaction a lieu en présence d'au moins un agent séquestrant de formule :

$$N—[CHR_1—CHR_2—O—(CHR_3—CHR_4—0)_n—R_5]_3 \qquad (I)$$

dans laquelle n est un nombre entier supérieur ou égal à 0 et inférieur ou égal à environ 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_m H_2m—\phi$ ou $C_m H_{2m+1}—\phi—$, où m est compris entre 1 et 12 ($1 \leq m \leq 12$).

Selon un mode de réalisation préférentiel de l'invention, on utilise un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer :

— la tris(oxa-3 butyl)amine de formule :
$N—(CH_2—CH_2—O—CH_3)_3$
— la tris(dioxa-3,6 heptyl)amine de formule :
$N—(CH_2—CH_2—O—CH_2—CH_2—O—CH_3)_3$
— la tris(trioxa-3,6,9 décyl)amine de formule :
$N—(CH_2—CH_2—O—CH_2—CH_2—O—CH_2—CH_2—O—CH_3)_3$
— la tris(dioxa-3,6 octyl)amine de formule :
$N—(CH_2—CH_2—O—CH_2—CH_2—O—C_2H_5)_3$
— la tris(trioxa-3,6,9 undécyl)amine de formule :
$N—(CH_2—CH_2—O—CH_2—CH_2—O—CH_2—CH_2—O—C_2H_5)_3$
— la tris(dioxa-3,6 nonyl)amine de formule :
$N—(CH_2—CH_2—O—CH_2—CH_2—O—C_3H_7)_3$
— la tris(trioxa-3,6,9 dodécyl)amine de formule :
$N—(CH_2—CH_2—O—CH_2—CH_2—O—CH_2—CH_2—O—C_3H_7)_3$
— la tris(dioxa-3,6 décyl)amine de formule :
$N—(CH_2—CH_2—O—CH_2—CH_2—O—C_4H_9)_3$
— la tris(trioxa-3,6,9 tridécyl)amine de formule :
$N—(CH_2—CH_2—O—CH_2—CH_2—O—CH_2—CH_2—O—C_4H_9)_3$
— la tris(tétra-oxa-3,6,9,12 tridécyl)amine de formule :
$N—[CH_2—CH_2—O—(CH_2—CH_2—O)_3—CH_3]_3$
— la tris(hexa-oxa-3,6,9,12,15,18 nonadécyl)amine de formule :
$N—[CH_2—CH_2—O—(CH_2—CH_2—O)_5—CH_3]_3$
— la tris(dioxa-3,6 méthyl-4 heptyl)amine de formule :
$N—[CH_2—CH_2—OCH—(CH_3)—CH_2—O—CH_3]_3$
— la tris(dioxa-3,6 diméthyl-2,4 heptyl)amine de formule :
$N—[CH_2—CH—(CH_3)—OCH(CH_3)—CH_2—O—CH_3]_3$.

Les amines utilisées dans le procédé selon l'invention sont connues en tant que telles dans l'art antérieur. C'est ainsi que le brevet français 1.302.365 cite l'obtention des amines tertiaires $N—(CH_2—CH_2—O—CH_3)_3$ et $N—(CH_2—CH_2—O—CH_2—CH_2—O—CH_3)_3$ comme sous produits de la

synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1.302.365 précité simultanément aux amines utilisées dans le procédé objet de la présente demande est totalement étranger au domaine de l'invention.

Les fluorobenzonitriles plus particulièrement concernés par le procédé selon l'invention ont pour formule générale :

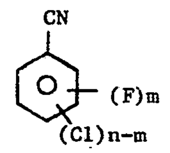

dans laquelle n et m sont des nombres entiers supérieurs ou égaux à 1 et inférieurs ou égaux à 5.

Ils sont préparés à partir des chlorobenzonitriles de formule générale :

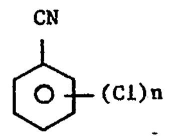

où n a la signification précédente.

Les fluorobenzonitriles encore plus particulièrement concernés par la présente invention sont ceux pour lesquels n et m sont supérieurs ou égaux à 1 et inférieurs ou égaux à 3 et où les atomes de fluor sont en position ortho et/ou para.

On peut citer : le fluoro-2 benzonitrile, le fluoro-4 benzonitrile, le difluoro-2,6 benzonitrile, le difluoro-2,4 benzonitrile, le fluoro-2 chloro-6 benzonitrile, le fluoro-4 chloro-3 benzonitrile, le fluoro-2 chloro-3 benzonitrile qui sont obtenus respectivement à partir du chloro-2 benzonitrile, chloro-4 benzonitrile, dichloro-2,6 benzonitrile, dichloro-2,4 benzonitrile, dichloro-2,6 benzonitrile, dichloro-3,4 benzonitrile, dichloro-2,3 benzonitrile. Bien entendu, les chlorobenzonitriles mis en œuvre dans le cadre du procédé de l'invention peuvent comporter, en outre, des substituants inertes dans les conditions de la réaction comme les radicaux $CF_3$ et alkyle par exemple.

Les fluorures alcalins pouvant être mis en œuvre dans le cadre de la présente invention sont principalement le fluorure de potassium et/ou le fluorure de caesium et/ou rubidium. Les fluorures alcalins doivent être utilisés sous forme anhydre.

Le solvant est de préférence choisi parmi le groupe comprenant la N-méthylpyrrolidone, le diméthylsulfoxyde, le diméthylformamide et le sulfolane.

Le sulfolane est plus particulièrement préféré.

Le choix de l'agent séquestrant le plus adapté à la mise en œuvre du procédé selon l'invention se fait en tenant compte de la taille du cation alcalin du fluorure alcalin mis en œuvre. Plus la taille du cation sera importante, plus le nombre d'atomes d'oxygène contenus dans la molécule de l'agent séquestrant devra être élevé.

C'est ainsi que lorsque l'on utilisera le fluorure de potassium, on préférera utiliser comme agent séquestrant la tris(trioxa-3,6,9 décyl)amine et/ou la tris(trioxa-3,6,9 undécyl)amine.

Le procédé selon l'invention est de préférence mis en œuvre à une température comprise entre 180 °C et 250 °C, la température étant d'autant plus basse que le chlorobenzonitrile de départ contient plus d'atomes de chlore.

On préfère opérer à pression atmosphérique bien que des pressions supérieures ou inférieures à la pression atmosphérique ne sont pas exclues du domaine de l'invention.

On utilise l'agent séquestrant en quantité telle que le rapport molaire de l'agent séquestrant au fluorure alcalin est compris entre 0,005 et 0,2. Encore plus préférentiellement, ce rapport est compris entre 0,01 et 0,1.

La quantité de fluorure alcalin à utiliser dépend évidemment du nombre d'atome de chlore à substituer par du fluor. On opère de préférence avec un excès de 10 à 50 % molaire par rapport à la stœchiométrie.

On utilise de préférence une quantité de solvant tel que le rapport molaire du solvant au chlorobenzonitrile de départ est compris entre 1 et 50 et de préférence entre 2 et 20.

Les temps de réaction sont généralement compris entre 3 heures et 24 heures.

L'invention va être maintenant plus complètement décrite dans les exemples qui vont suivre. Ces exemples ne sauraient être interprétés comme limitant de façon quelconque l'invention.

## Exemple 1

Préparation du parafluorobenzonitrile à partir du parachlorobenzonitrile.

Dans un ballon tricol de 500 ml équipé d'un agitateur mécanique, d'un condenseur et d'un thermomètre, on introduit successivement :

— 250 g de sulfolane (2 moles) anhydre
— 13,7 g de parachlorobenzonitrile (0,1 mole)
— 6,4 g de fluorure de potassium anhydre (0,11 mole) et
— 4,6 g de tris(trioxa-3,6,9 décyl)amine (0,01 mole).

Le mélange est chauffé à 220 °C sous agitation pendant 24 H 00 puis refroidi et filtré.

L'analyse par chromatographie gazeuse de la phase organique indique 72 % de parafluorobenzonitrile et 28 % de parachlorobenzonitrile non transformé.

Essai comparatif

En l'absence de tris(trioxa-3,6,9 décyl)amine dans les mêmes conditions opératoires, après 24 H 00 à 220 °C, la composition du mélange est 51 % de parafluorobenzonitrile et 49 % de parachlorobenzonitrile non transformé.

Exemple 2

Préparation du difluoro-2,6 benzonitrile à partir du dichloro-2,6 benzonitrile.

Dans un ballon tricol de 0,5 litre muni d'une agitation, d'un condenseur et d'un thermomètre, on introduit :

— 260 g de sulfolane anhydre
— 63,8 g (1,1 mole) de fluorure de potassium sec
— 68,7 g (0,4 mole) de dichloro-2,6 benzonitrile et
— 4,5 g (0,01 mole) de tris(trioxa-3,6,9 décyl)amine.

On chauffe sous agitation jusqu'à 210 °C. Au bout de 3 H 30 de réaction à 210 °C, l'analyse chromatographique du mélange réactionnel donne la composition suivante :

— dichloro-2,6 benzonitrile = 1 %
— fluoro-2 chloro-6 benzonitrile = 17,4 %
— difluoro-2,6 benzonitrile = 81,6 %

et après 5 H 30 de réaction à 210 °C, la composition est :

— dichloro-2,6 benzonitrile < 0,1 %
— fluoro-2 chloro-6 benzonitrile = 4,7 %
— difluoro-2,6 benzonitrile = 95,3 %

Les produits sont isolés selon la méthode traditionnelle.

Essai comparatif

En opérant dans les mêmes conditions mais sans tris(trioxa-3,6,9 décyl)amine, au bout de 3 H 30 de réaction, la composition est :

— dichloro-2,6 benzonitrile = 3 %
— fluoro-2 chloro-6 benzonitrile = 30 %
— difluoro-2,6 benzonitrile = 67 %

Au bout de 5 H 30 de réaction, la composition est :

— dichloro-2,6 benzonitrile < 0,1 %
— fluoro-2 chloro-6 benzonitrile = 15 %
— difluoro-2,6 benzonitrile = 85 %

Exemple 3

Préparation du difluoro-2,6 benzonitrile à partir du dichloro-2,6 benzonitrile.

Dans l'appareillage décrit à l'exemple 2, on introduit :
— 215 g de N-méthyle, 2 pyrrolidone (2,17 moles)
— 68,75 g de dichloro-2,6 benzonitrile (0,40 mole)

— 63,8 g de KF (1,1 mole)
— 4,5 g de tris(trioxa-3,6,9 décyl)amine (0,01 mole).

On chauffe sous agitation jusqu'à 210 °C.
Au bout de 3 H 30 de réaction, l'analyse chromatographique du mélange obtenu donne :

— dichloro-2,6 benzonitrile : 2 %
— fluoro-2 chloro-6 benzonitrile : 32 %
— difluoro-2,6 benzonitrile : 66 %

Au bout de 5 H 30 de réaction, on observe la composition suivante :

— dichloro-2,6 benzonitrile : 0,3 %
— fluoro-2 chloro-6 benzonitrile : 16 %
— difluoro-2,6 benzonitrile : 83,7 %

Essai comparatif

En opérant comme ci-dessus mais sans tris(trioxa-3,6,9 décyl)amine, on a au bout de 3 H 30 :

— dichloro-2,6 benzonitrile : 5 %
— fluoro-2 chloro-6 benzonitrile : 45 %
— difluoro-2,6 benzonitrile : 50 %

Au bout de 5 H 30, la composition est la suivante :
— dichloro-2,6 benzonitrile : 2 %
— fluoro-2 chloro-6 benzonitrile : 28 %
— difluoro-2,6 benzonitrile : 70 %

**Revendications**

1. Procédé de préparation de fluorobenzonitriles par réaction des chlorobenzonitriles correspondants avec au moins un fluorure alcalin en milieu solvant aprotique caractérisé en ce que la réaction a lieu en présence d'au moins un agent séquestrant de formule :

$$N{-}[CHR_1{-}CHR_2{-}O{-}(CHR_3{-}CHR_4{-}O)_n{-}R_5]_3 \qquad (I)$$

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\phi$ ou $C_mH_{2m+1}-\phi-$, où m est compris entre 1 et 12 ($1 \leq m \leq 12$).

2. Procédé selon la revendication 1 caractérisé en ce que dans la formule (I) $R_1$, $R_2$, $R_3$ et $R_4$ représentent un atome d'hydrogène ou un radical méthyle.

3. Procédé selon la revendication 1 caractérisé en ce que dans la formule (I) n est un nombre entier supérieur ou égal à 0 et inférieur ou égal à 6.

4. Procédé selon la revendication 1 caractérisé en ce que dans la formule (I) $R_5$ représente un radical alkyle ayant de 1 à 4 atomes de carbone.

5. Procédé selon la revendication 1 caractérisé en ce que dans la formule (I) $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

6. Procédé selon la revendication 5 caractérisé en ce que l'agent séquestrant de formule (I) est la tris(trioxa-3,6,9 décyl)amine, de formule :

$$N{-}(CH_2{-}CH_2{-}O{-}CH_2{-}CH_2{-}O{-}CH_2{-}CH_2{-}O{-}CH_3)_3$$

7. Procédé selon la revendication 5 caractérisé en ce que l'agent séquestrant de formule I est la tris(trioxa-3,6,9 undécyl)amine, de formule :

$$N{-}(CH_2{-}CH_2{-}O{-}CH_2{-}CH_2{-}O{-}CH_2{-}CH_2{-}O{-}C_2H_5)_3$$

8. Procédé selon l'une quelconque des revendications précédentes caractérisé en ce que l'agent séquestrant est utilisé en quantité telle que le rapport molaire de l'agent séquestrant au fluorure alcalin

est compris entre 0,005 et 0,2.

9. Procédé selon la revendication 8 caractérisé en ce que le rapport est compris entre 0,01 et 0,1.

## Claims

1. Process for the preparation of fluorobenzonitriles by reaction of the corresponding chlorobenzonitriles with at least one alkali metal fluoride in an aprotic solvent medium, characterised in that the reaction takes place in the presence of at least one sequestering agent of the formula :

$$N{-}[CHR_1{-}CHR_2{-}O{-}(CHR_3{-}CHR_4{-}O)_n{-}R_5]_3 \quad (I)$$

in which n is an integer which is greater than or equal to 0 and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical $-C_mH_{2m}-\phi$ or $C_mH_{2m+1}-\phi-$, in which m is between 1 and 12 ($1 \leq m \leq 12$).

2. Process according to Claim 1, characterised in that, in the formula (I), $R_1$, $R_2$, $R_3$ and $R_4$ represent a hydrogen atom or a methyl radical.

3. Process according to Claim 1, characterised in that, in the formula (I), n is an integer which is greater than or equal to 0 and less than or equal to 6.

4. Process according to Claim 1, characterised in that, in the formula (I), $R_5$ represents an alkyl radical having from 1 to 4 carbon atoms.

5. Process according to Claim 1, characterised in that, in the formula (I), $R_1$, $R_2$, $R_3$ and $R_4$, which are identical or different, represent a hydrogen atom or a methyl radical, n is an integer which is greater than or equal to 0 and less than or equal to 6, and $R_5$ represents an alkyl radical having from 1 to 4 carbon atoms.

6. Process according to Claim 5, characterised in that the sequestering agent of the formula (I) is tris-(3,6,9-trioxadecyl)-amine of the formula :

$$N{-}(CH_2{-}CH_2{-}O{-}CH_2{-}CH_2{-}O{-}CH_2{-}CH_2{-}O{-}CH_3)_3$$

7. Process according to Claim 5, characterised in that the sequestering agent of the formula I is tris-(3,6,9-trioxaundecyl)-amine of the formula :

$$N{-}(CH_2{-}CH_2{-}O{-}CH_2{-}CH_2{-}O{-}CH_2{-}CH_2{-}O{-}C_2H_5)_3$$

8. Process according to any one of the preceding claims, characterised in that the sequestering agent is used in an amount such that the molar ratio of the sequestering agent to the alkali metal fluoride is between 0.005 and 0.2.

9. Process according to Claim 8, characterised in that the ratio is between 0.01 and 0.1.

## Ansprüche

1. Verfahren zur Herstellung von Fluorbenzonitrilen durch Umsetzung von entsprechenden Chlorbenzonitrilen mit wenigstens einem Alkalifluorid in einem aprotischen Lösungsmittel, dadurch gekennzeichnet, daß die Reaktion in gegenwart wenigstens eines Sequestrierungsmittels der allgemeinen Formel

$$N{-}[CHR_1{-}CHR_2{-}O{-}(CHR_3{-}CHR_4){-}O)_n{-}R_5]_3 \quad (I)$$

erfolgt, in der n eine ganze Zahl größer oder gleich 0 und kleiner oder gleich 10 ($0 \leq n \leq 10$) ist, $R_1$, $R_2$, $R_3$, $R_4$ gleich oder verschieden ein Wasserstoffatom oder einen Alkylrest mit 1 bis 4 Kohlenstoffatomen und $R_5$ ein Alkyl- oder Cycloalkylrest mit 1 bis 12 Kohlenstoffatomen, ein Phenylrest oder einen Rest $-C_mH_{2m}-\phi$ oder $C_mH_{2m+1}-\phi-$ bedeuten, in dem m 1 bis 12 ist ($1 \leq m \leq 12$).

2. Verfahren nach Anspruch 1, dadurch gekennzeichnet, daß in der Formel (I) $R_1$, $R_2$, $R_3$ und $R_4$ ein Wasserstoffatom oder einen Methylrest bedeuten.

3. Verfahren nach Anspruch 1, dadurch gekennzeichnet, daß in der Formel (I) n eine ganze Zahl größer oder gleich 0 und kleiner oder gleich 6 ist.

4. Verfahren nach Anspruch 1, dadurch gekennzeichnet, daß in der Formel (I) $R_5$ ein Alkylrest mit 1 bis 4 Kohlenstoffatomen ist.

5. Verfahren nach Anspruch 1, dadurch gekennzeichnet, daß in der Formel (I) $R_1$, $R_2$, $R_3$ und $R_4$ gleich oder verschieden ein Wasserstoffatom oder einem Methylrest bedeuten und n eine ganze Zahl größer oder gleich 0 und kleiner oder gleich 6 ist und $R_5$ einen Alkylrest mit 1 bis 4 Kohlenstoffatomen

bedeutet.

6. Verfahren nach Anspruch 5, dadurch gekennzeichnet, daß das Sequestrierungsmittel der Formel (I) Tris(3,6,9-trioxadecyl)amin der Formel

$$N—(CH_2—CH_2—O—CH_2—CH_2—O—CH_2—CH_2—O—CH_3)_3$$

ist.

7. Verfahren nach Anspruch 5, dadurch gekennzeichnet, daß das Sequestrierungsmittel der Formel (I) Tris(3,6,9-trioxaundecyl)amin der Formel

$$N—(CH_2—CH_2—O—CH_2—CH_2—O—CH_2—CH_2—O—C_2H_5)_3$$

ist.

8. Verfahren nach irgendeinem der vorangegangenen Ansprüche, dadurch gekennzeichnet, daß das Sequestrierungsmittel in einer solchen Menge verwendet wird, daß das Molverhältnis des Sequestrierungsmittels zum Alkalifluorid 0,005 bis 0,2 beträgt.

9. Verfahren nach Anspruch 8, dadurch gekennzeichnet, daß das Verhältnis 0,01 bis 0,1 beträgt.